# EUROPÄISCHE PATENTSCHRIFT

(11) **EP 1 013 326 B1**
(45) Veröffentlichungstag und Bekanntmachung des Hinweises auf die Patenterteilung: **08.06.2005**
(21) Anmeldenummer: 99125602.5
(22) Anmeldetag: 22.12.1999
(51) Int. Cl.: B01D 21/32, B01D 21/00, G01N 1/14, G01N 33/18, B01D 21/24

(54) **Vorrichtung zur Bestimmung des Feststoffanteiles in Klärwasser**
Device for determining the solids content in clarified water
Dispositif de détermination de la quantité de solide dans une eau clarifiée

(30) Priorität: 23.12.1998 DE 19859726
(43) Veröffentlichungstag der Anmeldung: 28.06.2000
(73) Patentinhaber: Hach Lange GmbH, 14163 Berlin (DE)
(72) Erfinder: Battefeld, Manfred, 40211 Düsseldorf (DE); Bringsken, Peter, 47239 Duisburg (DE); Kussmann, Michael, 40476 Düsseldorf (DE)
(74) Vertreter: Selting, Günther, Dipl.-Ing.

(56) Entgegenhaltungen:
- EP-A- 0 777 117
- DE-A- 3 706 458
- DE-A- 19 544 851
- DE-U- 29 607 783
- GB-A- 2 085 843
- US-A- 5 154 835

## Beschreibung

Die Erfindung bezieht sich auf eine Vorrichtung zur Bestimmung des Feststoffanteiles in Klärwasser eines Klärbeckens.

Bei Kläranlagen ist die Bestimmung des Feststoffanteiles in dem Klärwasser ein gängiges Verfahren, um wichtige Parameter des Klärwassers zu ermitteln. So wird beispielsweise in Schlamm-Belebungsbecken von Kläranlagen der Schlammvolumenanteil bestimmt, um die Menge und Qualität des aktiven Belebtschlammes beurteilen und steuern zu können. Gemäß einem in DIN 38414, Teil 10, definierten Meßverfahren wird zur Bestimmung des Schlammvolumens bei einer Klärwasserprobe von 1,0 l nach 30 Minuten die Höhe des sich in einem Sedimentations-Behälter unten abgesetzten Schlammvolumens gemessen und daraus das Schlammvolumen bezogen auf 1,0 Liter errechnet. Zur Vornahme dieser Bestimmung ist eine Vorrichtung bekannt, deren Sedimentations-Behälter außerhalb des Klärbeckens angeordnet ist. Als Klärwasser-Entnahmeeinrichtung dient eine Pumpe, die über eine Saugleitung das Klärwasser aus dem Klärbecken in den Behälter saugt. Eine Feststoffpegel-Meßvorrichtung in Form einer Infrarot-Lichtschranke bestimmt nach einer festgelegten Meßzeit den Pegel des abgesetzten Schlammes. Durch den Pumpendruck, den langen Förderweg und ggf. den Pumpenrotor können die Schlammflocken in ihrer Struktur und Größe verändert werden, so daß die Schlammvolumen-Bestimmung nicht die tatsächlichen Verhältnisse innerhalb des Klärbeckens wiederspiegelt. Die lange Förderleitung kann durch den Schlamm und/oder Einfrieren verstopfen. Ferner ist für die Förderung je nach Förderweglänge und -höhe ein nicht unerheblicher Stromverbrauch für die Förderpumpe anzusetzen.

Aufgabe der Erfindung ist es, eine Vorrichtung und ein Verfahren zur genaueren Bestimmung des Feststoffanteiles in Klärwasser zu schaffen.

Diese Aufgabe wird erfindungsgemäß durch die Merkmale der Ansprüche 1 und 15 gelöst.

Die erfindungsgemäße Vorrichtung nach Anspruch 1 weist als Sedimentations-Behälter einen in Klärwasser eintauchbaren Tauchbehälter auf, der in dem Klärbecken installierbar ist, wobei die Entnahmevorrichtung eine an dem Tauchbehälter vorgesehene Einlauföffnung aufweist. Mit dem Sedimentations-Tauchbehälter wird der Meßort also in das Klärwasser des Klärbeckens verlegt, so daß Kärwasserleitungen zu dem Sedimentations-Behälter gänzlich entfallen können. Die Verhältnisse in dem Sedimentations-Behälter sind also nahezu identisch mit denen des Klärbeckens.

So weist der in das Klärbecken eingetauchte Tauchbehälter und das darin befindliche Klärwasser stets die gleiche Temperatur auf, wie das Klärbecken-Klärwasser außerhalb des Tauchbehälters. Dadurch wird das Meßergebnis verfälschende Konvexion innerhalb des Sedimentations-Behälters vermieden.

Durch die Verlegung des Meßortes in das Klärbecken und durch den Wegfall der Klärwasserleitungen entfällt die Gefahr von Verstopfungen oder Einfrieren. Durch den Wegfall eines Pumpenunterdruckes und eines Pumpenrotors entfällt auch eine mechanische Veränderung der Feststoffanteile, so daß die Messung in dem Sedimentations-Tauchbehälter exakt die Verhältnisse in dem Klärbecken wiederspiegelt. Durch den Wegfall von Förderpumpen für die Klärwasserförderung wird auch der Energieaufwand zum Betrieb der Vorrichtung erheblich reduziert. Ferner wirkt sich die Vereinfachung der Gesamtkonstruktion der Meßvorrichtung auch bei ihrer Herstellung kostensenkend aus. Durch den Wegfall der Klärwasserleitungen wird ferner ein schnelleres Befüllen und Entleeren des Behälters realisiert, wodurch wiederum kürzere Meßzyklen erreicht werden, so daß die nachfolgende Regelung der Klärwasserqualität verbessert wird.

Gemäß einer bevorzugten Ausgestaltung weist der Tauchbehälter eine Entlüftungsvorrichtung auf, die beim Zuführen von Klärwasser durch die Einlauföffnung in den Tauchbehälter geöffnet ist. Der luftgefüllte und in das Klarwässer eingetauchte Tauchbehälter wird über die geöffnete Entlüftungsvorrichtung zur Atmosphäre hin entlüftet, so daß das Klärwasser in den Tauchbehälter einfließen kann. Eine Pumpe für das Befüllen des Sedimentations-Tauchbehälters ist also nicht erforderlich, sondern nur ein einfaches Ventil zum Öffnen und Schließen der Entlüftungsvorrichtung. Ist der Tauchbehälter ausreichend mit Klärwasser gefüllt, so schließt die Entlüftungsvorrichtung wieder. Damit ist ein einfaches Mittel zum Befüllen des Tauchbehälters geschaffen.

Vorzugsweise weist der Tauchbehälter eine Druckluftversorgung auf, durch die der Tauchbehälter zum Ausstoßen des Klärwassers durch eine Auslaßöffnung mit Überdruck beaufschlagbar ist. Zum Entleeren des eingetauchten Tauchbehälters wird also Druckluft in den Tauchbehälter gegeben, die nach Abschluß der Messung das in Feststoffphase und Klarphase getrennte Klärwasser durch eine Auslaßöffnung wieder in das Klärbecken zurück drückt. Zum Betrieb der Feststoffanteil-Bestimmungsvorrichtung wird also allenfalls eine einzige Pumpe benötigt, die in dem Tauchbehälter lediglich einen geringen Überdruck erzeugen muß. Dadurch ist der mechanische Aufwand zum Füllen und Befüllen des Tauchbehälters sehr gering und wird eine preiswerte Herstellung und ein zuverlässiger Betrieb gewährleistet.

Gemäß einer bevorzugten Ausgestaltung sind die Einlauföffnung und die Auslaßöffnung eine einzige gemeinsame Öffnung im Bereich des Tauchbehälterbodens. Der Tauchbehälter weist also nur eine einzige Öffnung auf, die sowohl zum Befüllen als auch zum Leeren des Tauchbehälters mit Klärwässer benutzt wird. Auch dies stellt eine Vereinfachung des Sedimentationsbehälters dar, die weniger Herstellungsaufwand erfordert und größere Zuverlässigkeit bewirkt.

Vorzugsweise ist der Tauchbehälter doppelt kegelig ausgebildet, d.h. er ist durch zwei mit ihren Grundebenen aneinanderliegende Kegelstumpfe zu einem Doppelkegel zusammengesetzt. Der obere Kegelteil ist länger und hat einen geringeren Kegelwinkel als der untere Kegelteil, der kürzer ist und einen größeren Kegelwinkel aufweist als der obere. Der obere Kegelteil weitet sich nach unten hin stetig auf, wodurch, wie Versuche ergeben haben, das Absinken von Feststoffen erheblich beschleunigt wird. Auf diese Weise wird die Dauer eines Meßzyklus erheblich verringert. Der untere Kegelteil verjüngt sich nach unten hin, d.h. der Querschnitt des Tauchbehälters vergrößert sich stetig nach oben hin. Kleine sich in dem unteren Kegel absetzende Feststoffmengen bewirken daher zunächst relativ große Höhenänderungen der abgesetzten Feststoffphase, so daß die Meßgenauigkeit bei geringen abgesetzten Feststoffmengen deutlich erhöht wird. Auf diese Weise läßt sich eine über einen sehr weiten Bereich konstante relative Meßgenauigkeit realisieren.

In einer bevorzugten Ausgestaltung weist die Meßvorrichtung einen Meßaufnehmer auf, der innerhalb des Tauchbehälters angeordnet ist. Durch die Anordnung des Meßaufnehmers innerhalb, nicht außerhalb, des Tauchbehälters ist kein zweiter Behälter zum Schutz der Meßvorrichtung außerhalb des Sedimentations-Behälters erforderlich.

Gemäß einer bevorzugten Ausgestaltung ist der Meßaufnehmer eine im wesentlichen über die gesamte Höhe vertikal in dem Tauchbehälter angeordnete Zeilenkamera. Mit Hilfe der Zeilenkamera und ggf. einer entsprechenden Lichteinleitung kann der Feststoffpegel in dem Sedimentations-Tauchbehälter kontinuierlich gemessen werden. Störanfällige mechanische Elemente sind nicht vorhanden. Die Zeilenkamera kann beispielsweise in einem transparenten Rohr angeordnet oder in transparenten Kunststoff vergossen in der Längsachse des Tauchbehälters angeordnet sein. Eine Beleuchtungsvorrichtung kann parallel dazu angeordnet sein. Alternativ dazu kann der Meßaufnehmer zur Messung des Feststoffpegels auch als Ultraschall-Höhenmeßvorrichtung ausgebildet sein. Dies hat den Vorteil, daß der Meßaufnehmer oberhalb des Tauchbehälters angeordnet ist, so daß keine Abdichtungs- und Korrosionsprobleme auftreten können.

Gemäß einer bevorzugten Ausgestaltung weist die Meßvorrichtung eine Auswertevorrichtung auf, die entfernt von dem Meßaufnehmer außerhalb des Klärbeckens installierbar ist. Die von dem Meßaufnehmer erzeugten Meßwerte werden, ggf. durch einen Wandler kodiert und/oder durch einen Verstärker verstärkt, von dem Tauchbehälter über entsprechende Meßleitungen oder drahtlos über Funk der Auswertevorrichtung übermittelt, die die Meßergebnisse zur weiteren Verwendung aufbereitet.

Gemäß einer bevorzugten Ausgestaltung wird der Feststoffpegel kontinuierlich durch den Meßaufnehmer erfaßt, so daß die Auswertevorrichtung durch Bewertung des zeitlichen Verlaufes des zunächst ansteigenden und dann wieder sinkenden Feststoffpegels einen voraussichtlichen Feststoffpegel-Endwert extrapolieren kann. Wie Versuche ergeben haben, läßt sich der Feststoffpegel-Endwert bereits lange vor dem vollständigen Absetzen aller Feststoffanteile des Klärwassers bestimmen, indem die Absetzrate ermittelt und mit abgespeicherten Werten aus einer Bibliothek verglichen wird. Die Absetzrate kann beispielsweise bei der Schlammvolumenmessung eine wertvolle Information über die Qualität von Belebtschlamm in einem Belebungsbecken liefern. Durch die genaue Ermittlung der Absetzrate und durch die Extrapolation des Feststoffpegel-Endwertes werden wertvolle Informationen zur Steuerung von Zuständen in Klärbecken mit hoher Genauigkeit bei gleichzeitig kurzen Meßzyklen geliefert.

Vorzugsweise weist die Tauchbehälter-Innenwand eine geringe Haftung aufweisende schmutzabweisende Beschichtung auf. Dadurch wird gewährleistet, daß beim Entleeren des Tauchbehälters keine Feststoffanteile an den Gehäusewänden hängenbleiben, so daß die folgende Messung nicht verfälscht wird. Zur Reinigung der Tauchbehälter-Innenwände kann alternativ oder ergänzend dazu eine Wischvorrichtung und/oder ein Ultraschallgenerator zum Entfernen nach der Behälterentleerung zurückgebliebener Feststoffpartikel eingesetzt werden.

In einer bevorzugten Ausgestaltung kann eine Hubvorrichtung vorgesehen sein, durch die der Tauchbehälter während des Trennens des zugeführten Klärwassers aus dem Klärwasser des Klärbeckens heraushebbar ist. Der Tauchbehälter wird also nur zum Befüllen in das Klärwasser des Klärbeckens eingetaucht und nach erfolgter Befüllung aus dem Klärwasser herausgehoben. Dadurch werden durch das Klärbecken-Klärwasser induzierte Vibrationen während der Absetzphase vermieden, wodurch das Meßergebnis ggf. genauer ist.

Das erfindungsgemäße Verfahren nach Anspruch 15 zur Bestimmung des Feststoffanteiles Klärwasser eines Klärbeckens weist die folgenden Verfahrensschritte auf:
Eintauchen eines Sedimentations-Tauchbehälters in das Klärbecken unterhalb des Klärwasserspiegels,
Entnahme von Klärwasser aus dem Klärbecken und Zuführen des Klärwassers in den eingetauchten Tauchbehälter,
Trennen der Feststoffphase von der Klarphase des zugeführten Klärwassers in dem Tauchbehälter durch Senkung,
Messen des Feststoffphasen-Pegels in dem Tauchbehälter, und
Bestimmung des Feststoffanteiles des Klärwassers anhand des gemessenen Feststoffphasen-Pegels.

Zum Auffüllen von Klärwasser wird der Sedimentations-Tauch-behälter in das Klärbeckenwasser eingetaucht und geöffnet, so daß das Klärwasser aus dem Klärbecken direkt und auf kürzestem Weg in den Sedimentations-Behälter einfließen kann. Durch den Wegfall von Leitungen für den Transport des Klärwassers aus dem Klärbecken in den Sedimentations-Behälter wird das Verfahren erheblich vereinfacht und beschleunigt.

Im folgenden wird unter Bezugnahme auf die Zeichnungen ein Ausführungsbeispiel der Erfindung näher erläutert.

Es zeigen:
- Fig. 1: eine in einem Kläranlagen-Klärbecken installierte Bestimmungsvorrichtung,
- Fig. 2: den Sedimentations-Behälter der Bestimmungsvorrichtung der Fig. 1 im Längsschnitt beim Befüllen,
- Fig. 3: den Sedimentations-Behälter während der Absetzphase,
- Fig. 4: den Sedimentations-Behälter am Ende der Absetzphase, und
- Fig. 5: den Sedimentations-Behälter beim Entleeren nach Abschluß eines Meßzyklus.

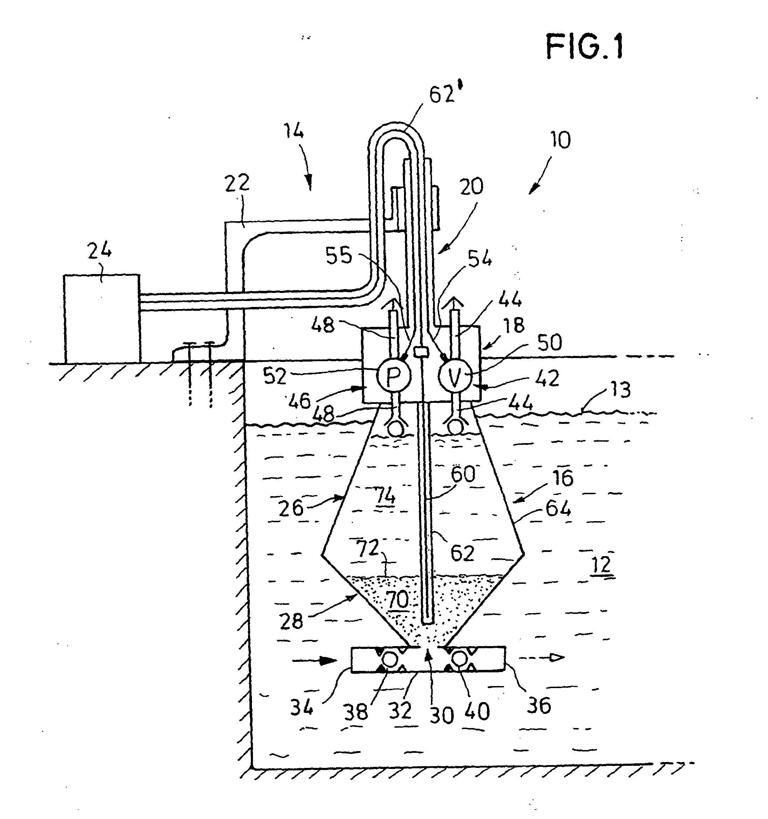

In den Figuren 1 bis 5 ist ein Klärbecken 10 einer Kläranlage dargestellt, das im vorliegenden Fall ein Schlammbelebungsbecken ist. In dem Schlammbelebungsbecken 10 wird mit Hilfe von sogenanntem Belebtschlamm das Klärwasser biologisch gereinigt. In einem nachfolgenden Verfahrensschritt wird das Klärwasser in einem Nachklärbecken von dem Belebtschlamm getrennt, indem man den Belebtschlamm absinken läßt und das Belebtschlammfreie Klärwasser abführt. Für diese Nachklärung in dem Nachklärbecken ist ein gutes Absetzverhalten des Belebtschlammes erforderlich. Die Belebtschlammqualität kann in dem Belebungsbecken 10 durch Steuerung der Belebtschlammkonzentration optimiert werden. Die Qualität des Belebtschlammes in dem Klärwasser 12 des Belebtschlamm-Klärbeckens 10 wird durch Messung des Schlammabsetzvolumenanteiles ermittelt.

Zur Bestimmung des Feststoffanteiles, also des Belebtschlammanteiles in dem Klärwasser 12 des Klärbeckens 10 ist eine Bestimmungsvorrichtung 14 an dem Klärbecken 10 vorgesehen, durch die der Feststoffanteil annähernd kontinuierlich bestimmt wird. Die Bestimmungsvorrichtung 14 besteht im wesentlichen aus einem Sedimentations-Tauchbehälter 16 mit aufgesetztem Schutzgehäuse 18 und mit einem Führungsrohr 20. Über einen das Führungsrohr 20 haltenden Halterahmen 22 wird der Sedimentations-Tauchbehälter 16 starr und vibrationsfrei in seiner Position innerhalb des Klärbeckens 10 knapp unterhalb des Klärwasser-Spiegels 13 fixiert. Außerhalb des Klärbeckens 10 ist eine Auswerte- und Steuervorrichtung 24 zum Steuern des Meßvorganges und Auswerten der Meßergebnisse vorgesehen.

Der Sedimentations-Tauchbehälter 16 ist als Doppelkegel ausgebildet, wobei der obere Kegelteil 26 sich nach unten hin mit einem Kegelwinkel von ungefähr 15° erweitert und der untere Kegelteil 28 sich mit einem Kegelwinkel von ungefähr 45° nach unten hin verengt. Am unteren Ende des unteren Kegelteils 28 des Sedimentations-Tauchbehälters 16 ist eine Einlauf-Auslaßöffnung 30 vorgesehen, an die ein waagerecht angeordnetes Rohr 32 mit einem offenen Zulaufende 34 und einem offenen Ablaufende 36 angeschlossen ist. In dem Rohr 32 sind zwei Rückschlagventile 38,40 in gleicher Flußrichtung wirkend angeordnet, so daß das eine offene Rohrende 34 einen Klärwassereinlauf und das andere offene Rohrende 36 einen Klärwasserauslauf des Tauchbehälters 16 bildet.

Oberhalb des Tauchbehälters 16 ist als Entlüftungsvorrichtung 42 eine in den Tauchbehälter 16 mündende Luftleitung 44 und als Druckluftversorgung 46 eine weitere Luftleitung 48 vorgesehen. Im Verlauf der Entlüftungsleitung 44 ist ein elektrisch steuerbares Entlüftungsventil 50 angeordnet. Im Verlauf der Druckluftversorgungsleitung 48 ist eine Druckluft erzeugende elektrisch betriebene Pumpe 52 angeordnet. Die beiden Leitungen 44,48 sind jeweils an ihren in den Sedimentations-Tauchbehälter 16 mündenden Enden mit Schwimmerventilen versehen, die bei Überschreiten eines maximalen Klärwasserstandes in dem Tauchbehälter 16 die Leitungsöffnungen verschließen, so daß kein Klärwasser aus dem Behälter 16 in die Leitungen 44,48 eindringen kann. Das Entlüftungsventil 50 und die Druckluftpumpe 52 werden jeweils über eine elektrische Versorgungsleitung 54,55 geschaltet bzw. betrieben.

In den Tauchbehälter 16 ist als Meßaufnehmer eine vertikal über annähernd die gesamte Länge sich erstreckende Zeilenkamera 60 in einem wasserdichten transparenten Gehäuse 62 angeordnet. Die Zeilenkamera 60 ist über mehrere Daten- und Steuerleitungen 62 mit der Auswerte- und Steuervorrichtung 24 verbunden. In dem Gehäuse 62 ist ferner eine nicht dargestellte Beleuchtungseinrichtung angeordnet, durch die der Innenraum des Tauchbehälters 16 während der Messungen beleuchtet wird.

Das Schutzgehäuse 18 oberhalb des Tauchbehälters 16 umgibt und schützt das obere Ende der Zeilenkamera 60 sowie die Druckluftversorgung 46 und die Entlüftungsvorrichtung 42 vor Fruchtigkeit und Verschmutzung.

Die Innenseite der Tauchbehälterwand 64 ist mit einer schmutzabweisenden Antihaftbeschichtung versehen, so daß beim Entleeren keine Rückstände an der Behälterinnenwand 64 haften bleiben. Ergänzend dazu kann ein Ultraschallgenerator zur Reinigung der Tauchbehälter-Innenwände 64 vorgesehen sein.

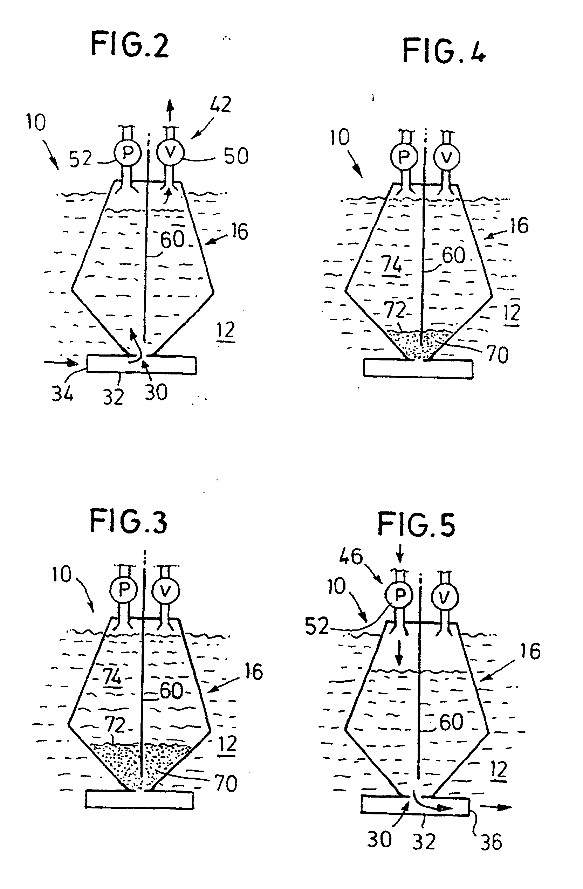

In den Figuren 2 bis 4 ist schrittweise ein Meßzyklus dargestellt: Zum Beginn eines Meßzyklus wird durch die Steuervorrichtung 24 das Entlüftungsventil 50 geöffnet, so daß der ausschließlich mit Luft gefüllte Tauchbehälter 16 zur Atmosphäre hin geöffnet wird. Dadurch kann das Klärwasser 12 aus dem Klärbecken 10 durch das Einlaßende 34 des Rohres 32 und die Einlaßöffnung 30 in den Tauchbehälter 16 einfließen, wie in Figur 2 dargestellt. Das Entlüftungsventil 50 wird wieder geschlossen, sobald durch die Zeilenkamera 60 das Erreichen des gewünschten Füllstandes detektiert wird. In den Figuren 3 und 4 ist der Absetzvorgang dargestellt, in dessen Verlauf sich die Feststoffanteile in einer Feststoffphase 70 im unteren Bereich des Tauchbehälters 16 zunächst aufschichten (Fig. 3) und anschließend zusammensacken (Fig. 4), und sich oberhalb des Feststoffpegels 72 eine Klarphase 74 bildet. Das Anwachsen und anschließende Zusammensacken der Feststoffphase 70 und damit das Steigen und anschließende Fallen des Feststoffpegels 72 wird kontinuierlich durch die Zeilenkamera 60 erfaßt und ausgewertet. Die Messung wird beendet, sobald das Abfallen des Feststoffpegels 72 einen festgelegten relativen Änderungswert unterschreitet. Auf diese Weise wird die Meßzeit an die jeweilige Absetzgeschwindigkeit der Feststoffe adaptiert, so daß die Meßzeit optimiert wird. Aus der Pegelhöhe und der Absetzzeit bis zum Erreichen eines vorbestimmten Wertes und einer auf andere Weise vorgenommenen Messung des Feststoffanteiles können Aussagen bezüglich der Qualität des Belebtschlammes gemacht werden und dementsprechend die Belebtschlammmenge und -qualität in dem Klärbecken 10 geregelt werden.

Nach Abschluß der Messung wird, wie in Figur 5 dargestellt, die Druckpumpe 52 eingeschaltet, durch die Druckluft in den Tauchbehälter 16 gepumpt wird, die wiederum das in zwei Phasen getrennte Klärwasser durch die Ausflußöffnung 30 in das Rohr 32 und durch das offene Rohrende 36 wieder zurück in das Klärbecken 10 drückt. Nach einer anschließenden Reinigung der Tauchbehälter-Innenwände durch eine erneute Spülung mit Klärwasser aus dem Klärbecken und einer Ultraschallreinigung ist ein Meßzyklus abgeschlossen und kann ein neuer Meßzyklus beginnen.

## Patentansprüche

1. Vorrichtung zur Bestimmung des Feststoffanteiles im Klärwasser (12) eines Klärbeckens (10), mit
einem Sedimentations-Behälter (16) zum Trennen der Feststoffphase (30) von der Klarphase (74) zugeführten Klärwassers,
einer Klärwasser-Entnahmeeinrichtung zur Entnahme von Klärwasser (12) aus dem Klärbecken (10), wobei das entnommene Klärwasser (12) dem Sedimentations-Behälter (16) zugeführt wird, und
einer Feststoffpegel-Meßvorrichtung (60) zur Bestimmung des Feststoffpegels (72) der in dem Sedimentationsbehälter (16) abgesetzten Feststoffphase (70) des Klärwassers,
**dadurch gekennzeichnet,**
**daß** der Sedimentations-Behälter (16) ein in Klärwasser (12) eintauchbarer Tauchbehälter (16) ist, der in dem Klärbecken (10) unterhalb des Klärwasserspiegels (13) installierbar ist, und
**daß** die Entnahmeeinrichtung eine an dem Tauchbehälter (16) vorgesehene Einlauföffnung (30) aufweist.

2. Vorrichtung nach Anspruch 1, **dadurch gekennzeichnet, daß** der Tauchbehälter (16) eine Entlüftungsvorrichtung (42) aufweist, die beim Zuführen von Klärwasser (12) durch die Einlauföffnung (30) in den Tauchbehälter (16) geöffnet ist.

3. Vorrichtung nach Anspruch 1 oder 2, **dadurch gekennzeichnet, daß** der Tauchbehälter (16) eine Druckluftversorgung (46) aufweist, durch die der Tauchbehälter (16) zum Ausstoßen des Klärwassers durch eine Auslaßöffnung (130) mit Überdruck beaufschlagt wird.

4. Vorrichtung nach einem der Ansprüche 1-3, **dadurch gekennzeichnet, daß** die Einlauföffnung und die Auslaßöffnung eine einzige gemeinsame Öffnung (30) im Bereich des Tauchbehälterbodens ist.

5. Vorrichtung nach einem der Ansprüche 1-4, **dadurch gekennzeichnet, daß** der Tauchbehälter (16) doppelt kegelig ausgebildet ist.

6. Vorrichtung nach einem der Ansprüche 1-5, **dadurch gekennzeichnet, daß** die Meßvorrichtung einen Meßaufnehmer (60) aufweist, der innerhalb des Tauchbehälters (16) angeordnet ist.

7. Vorrichtung nach einem der Ansprüche 1-6, **dadurch gekennzeichnet, daß** der Meßaufnehmer eine im wesentlichen über die gesamte Höhe vertikal in dem Tauchbehälter (16) angeordnete Zeilenkamera (60) ist.

8. Vorrichtung nach einem der Ansprüche 1-6, **dadurch gekennzeichnet, daß** der Meßaufnehmer eine Ultraschall-Höhenmeßvorrichtung ist.

9. Vorrichtung nach einem der Ansprüche 1-8, **dadurch gekennzeichnet, daß** die Meßvorrichtung eine Auswertevorrichtung aufweist, die entfernt von dem Meßaufnehmer (60) außerhalb des Klärbeckens (10) installierbar ist.

10. Vorrichtung nach einem der Ansprüche 6-9, **dadurch gekennzeichnet, daß** der Feststoffpegel (72) kontinuierlich durch den Meßaufnehmer (60) erfaßt wird und die Auswertevorrichtung (24) durch Bewertung des zeitlichen Verlaufes des Feststoffpegels einen voraussichtlichen Feststoffpegel-Endwert ermittelt.

11. Vorrichtung nach einem der Ansprüche 1-10, **dadurch gekennzeichnet, daß** die Tauchbehälter-Innenwand (64) eine geringe Haftung aufweisende schmutzabweisende Beschichtung aufweist.

12. Vorrichtung nach einem der Ansprüche 1-11, **dadurch gekennzeichnet, daß** eine Wischvorrichtung zur Reinigung der Tauchbehälter-Innenwände vorgesehen ist.

13. Vorrichtung nach einem der Ansprüche 1-12, **dadurch gekennzeichnet, daß** ein Ultraschallgenerator zur Reinigung der Tauchbehälter-Innenwände vorgesehen ist.

14. Vorrichtung nach einem der Ansprüche 1-13, **dadurch gekennzeichnet, daß** eine Hubvorrichtung vorgesehen ist, durch die der Tauchbehälter während des Trennens des zugeführten Klärwassers aus dem Klärwasser des Klärbeckens heraushebbar ist.

15. Verfahren zur Bestimmung des Feststoffanteiles in Klärwasser (12) eines Klärbeckens (10), mit den Schritten:
Eintauchen eines Sedimentations-Tauchbehälters (16) in das Klärbecken (10) unterhalb des Klärwasser-Spiegels (13),
Einlassen von Klärwasser (12) aus dem Klärbecken (10) in den eingetauchten Tauchbehälter (16),
Trennen der Feststoffphase (70) von der Klarphase (74) des zugeführten Klärwassers in dem Tauchbehälter (16) durch Absenkung,
Messen des Feststoffphasen-Pegels (72) in dem Tauchbehälter (16), und
Bestimmung des Feststoffanteiles des Klärwassers anhand des gemessenen Feststoffphasen-Pegels (72).

## Claims

1. A device for determining the solids content in the sewage water (12) of a clarification basin (10), comprising
a sedimentation container (16) for separating the solids phase (30) from the clear phase (74) of supplied sewage water,
a sewage water removal device for removing sewage water (12) from the clarification basin (10), the removed sewage water (12) being supplied to the sedimentation container (16), and
a solids level measuring device (60) for determining the solids level (72) of the solids phase (70) of the sewage water settled in the sedimentation container (16),
**characterized in**
**that** the sedimentation container (16) is an immersion container (16) immersible into sewage water (12) and adapted to be installed in the clarification basin (10) below the sewage water level (13), and
**that** the removal device comprises an inflow opening (30) provided on the immersion container (16).

2. The device according to claim 1, **characterized in that** the immersion container (16) comprises a ventilating means (42) which is opened during the supply of sewage water (12) through the inflow opening (30) into the immersion container (16).

3. The device according to claim 1 or 2, **characterized in that** the immersion container (16) comprises a pressurized air supply means (46) arranged to supply overpressure to the immersion container (16) for discharging the sewage water through an outlet opening (130).

4. The device according to any one of claims 1 - 3, **characterized in that** the inflow opening and the outlet opening comprise a sole common opening (30) in the region of the immersion container bottom.

5. The device according to any one of claims 1 - 4, **characterized in that** the immersion container (16) has a double-coned shape.

6. The device according to any one of claims 1 - 5, **characterized in that** the measuring device comprises a measurement pick-up (60) arranged internally of the immersion container (16).

7. The device according to any one of claims 1 - 6, **characterized in that** the measurement pick-up comprises a line camera (60) arranged vertically in the immersion container (16) substantially along the complete height.

8. The device according to any one of claims 1 - 6, **characterized in that** the measurement pick-up comprises an ultrasonic height measurement means.

9. The device according to any one of claims 1 - 8, **characterized in that** the measurement means comprises an evaluation means adapted to be installed remote from the measurement pick-up (60) externally of the clarification basin (10).

10. The device according to any one of claims 6 - 9, **characterized in that** the solids level (72) is continuously detected by the measurement pick-up (60) and that the evaluation means (24) obtains a prospective solids-level end value by evaluating the development over time of the solids level.

11. The device according to any one of claims 1 - 10, **characterized in that** the immersion container inner wall (64) is provided with a contamination-repelling coating with low adhesion.

12. The device according to any one of claims 1 - 11, **characterized in that** a wiper means is provided for cleaning the immersion container inner walls.

13. The device according to any one of claims 1 - 12, **characterized in that** an ultrasound generator is provided for cleaning the immersion container inner walls.

14. The device according to any one of claims 1 - 13, **characterized in that** a lifting means is provided for lifting the immersion container out of the sewage water of the clarification basin during the separation of the supplied sewage water.

15. A method for determining the solids content in the sewage water (12) of a clarification basin (10), comprising the following steps:
immersing a sedimentation immersion container (16) into the clarification basin (10) below the sewage water level (13),
causing sewage water (12) to flow from the clarification basin (10) into the immersed immersion container (16),
separating the solids phase (70) from the clear phase (74) of the supplied sewage water by draw-down,
measuring the solids phase level (72) in the immersion container (16), and
determining the solids content of the sewage water on the basis of the measured solids phase level (72).

## Revendications

1. Dispositif de détermination de la quantité de solide dans l'eau clarifiée (12) d'un bassin de décantation (10), comportant
une cuve de sédimentation (16) pour la séparation de la phase solide (30) de la phase clarifiée (74) de l'eau clarifiée amenée,
un dispositif de prélèvement d'eau clarifiée pour le prélèvement de l'eau clarifiée (12) hors du bassin de décantation (10), l'eau clarifiée (12) prélevée étant amenée à la cuve de sédimentation (16), et
un dispositif de mesure du niveau de solide (60) pour la détermination du niveau de solide (72) de la phase solide (70) de l'eau clarifiée, déposée dans la cuve de sédimentation (16),
**caractérisé en ce que**
la cuve de sédimentation (16) est une cuve à immersion (16) à immerger dans l'eau clarifiée (12), qui s'installe dans le bassin de décantation (10) en dessous de la surface (13) de l'eau clarifiée, et
le dispositif de prélèvement présente une ouverture d'entrée (30) prévue sur la cuve à immersion (16).

2. Dispositif selon la revendication 1, **caractérisé en ce que** la cuve à immersion (16) présente un dispositif de ventilation (42) qui est ouvert en cas d'amenée d'eau clarifiée (12), à travers l'ouverture d'entrée (30), dans la cuve à immersion (16).

3. Dispositif selon la revendication 1 ou 2, **caractérisé en ce que** la cuve à immersion (16) présente une alimentation en air comprimé (46) par laquelle la cuve à immersion (16) est exposée à une surpression pour évacuer l'eau clarifiée à travers une ouverture de sortie (130).

4. Dispositif selon l'une quelconque des revendications 1 à 3, **caractérisé en ce que** l'ouverture d'entrée et l'ouverture de sortie constituent une unique ouverture commune (30) dans la zone du fond de la cuve à immersion.

5. Dispositif selon l'une quelconque des revendications 1 à 4, **caractérisé en ce que** la cuve à immersion (16) est conçue en forme de double cône.

6. Dispositif selon l'une quelconque des revendications 1 à 5, **caractérisé en ce que** le dispositif de mesure présente un capteur de mesure (60) qui est disposé à l'intérieur de la cuve à immersion (16).

7. Dispositif selon l'une quelconque des revendications 1 à 6, **caractérisé en ce que** le capteur de mesure est une caméra à lignes (60) disposée sur toute la hauteur verticalement dans la cuve à immersion (16).

8. Dispositif selon l'une quelconque des revendications 1 à 6, **caractérisé en ce que** le capteur de mesure est un dispositif de mesure de la hauteur à ultrasons.

9. Dispositif selon l'une quelconque des revendications 1 à 8, **caractérisé en ce que** le dispositif de mesure présente un dispositif d'évaluation qui est à installer à distance du capteur de mesure (60) en dehors du bassin de décantation (10).

10. Dispositif selon l'une quelconque des revendications 6 à 9, **caractérisé en ce que** le niveau de solide (72) est détecté en continu par le capteur de mesure (60) et le dispositif d'évaluation (24) détermine, par évaluation de l'évolution du niveau de solide en fonction du temps, une valeur finale estimée du niveau de solide.

11. Dispositif selon l'une quelconque des revendications 1 à 10, **caractérisé en ce que** la paroi intérieure (64) de la cuve à immersion présente un revêtement antisalissure présentant une faible adhérence.

12. Dispositif selon l'une quelconque des revendications 1 à 11, **caractérisé en ce qu'**un dispositif d'essuyage est prévu pour le nettoyage des parois intérieures de la cuve à immersion.

13. Dispositif selon l'une quelconque des revendications 1 à 12, **caractérisé en ce qu'**un générateur d'ultrasons est prévu pour le nettoyage des parois intérieures de la cuve à immersion.

14. Dispositif selon l'une quelconque des revendications 1 à 13, **caractérisé en ce qu'**il est prévu un dispositif de levage au moyen duquel la cuve à immersion peut être soulevée hors de l'eau clarifiée du bassin de décantation pendant la séparation de l'eau clarifiée amenée.

15. Procédé de détermination de la quantité de solide dans de l'eau clarifiée (12) d'un bassin de décantation (10), comprenant les étapes consistant à
immerger une cuve à immersion de sédimentation (16) dans le bassin de décantation (10) en dessous de la surface (13) de l'eau clarifiée,
introduire de l'eau clarifiée (12) provenant du bassin de décantation (10) dans la cuve à immersion (16) immergée,
séparer la phase solide (70) de la phase clarifiée (74) de l'eau clarifiée amenée dans la cuve à immersion (16) par abaissement,
mesurer le niveau (72) de la phase solide dans la cuve à immersion (16), et
déterminer la quantité de solide de l'eau clarifiée à l'aide du niveau (72) mesuré de la phase solide.
